# FASCICULE DE BREVET EUROPEEN

(11) **EP 3 787 592 B1**
(45) Date de publication et mention de la délivrance du brevet: **30.08.2023**
(21) Numéro de dépôt: 19720686.5
(22) Date de dépôt: 03.05.2019
(51) Int. Cl.: A61K 8/49, A61Q 7/00, A61K 8/9789, A61P 17/14, A61K 36/74

(54) **ASSOCIATION D'EXTRAITS DE QUINQUINA ET DE LEONTOPODIUM ALPINUM ET DU SEL DE MANGANESE DE L'ACIDE CARBOXYLIQUE L-PYRROLIDONE DANS LE TRAITEMENT DE L'ALOPECIE**
KOMBINATION VON EXTRAKTEN VON CHINARINDE UND LEONTOPODIUM ALPINUM UND DES MANGANSALZES VON L-PYRROLIDONCARBONSÄURE ZUR BEHANDLUNG VON ALOPEZIE
COMBINATION OF EXTRACTS OF QUINQUINA AND OF LEONTOPODIUM ALPINUM AND OF THE MANGANESE SALT OF L-PYRROLIDONECARBOXYLIC ACID IN THE TREATMENT OF ALOPECIA

(30) Priorité: 04.05.2018 FR 1853867
(43) Date de publication de la demande: 10.03.2021
(73) Titulaire: Pierre Fabre Dermo-Cosmétique, 92100 Boulogne-Billancourt (FR)
(72) Inventeur: DAUNES-MARION, Sylvie, 31000 TOULOUSE (FR); LEVEQUE, Marguerite, 31400 TOULOUSE (FR); POIGNY, Stéphane, 31600 SAUBENS (FR); MANDEAU, Anne, 31200 TOULOUSE (FR)
(74) Mandataire: Regimbeau
(86) Numéro de dépôt international: PCT/EP2019/061445
(87) Numéro de publication internationale: WO 2019/211468

(56) Documents cités:
- EP-A1- 2 810 697
- FR-A1- 2 751 541
- FR-A1- 2 853 245

## Description

### DOMAINE TECHNIQUE

La présente invention concerne une nouvelle association d'un extrait de quinquina, du sel de manganèse de l'acide carboxylique L-pyrrolidone et d'un extrait de *Leontopodium alpinum,* ainsi que l'utilisation de ladite association dans le domaine capillaire plus particulièrement dans le traitement ou la prévention de l'alopécie.

### ETAT DE LA TECHNIQUE ANTERIEURE

Le soin des cheveux, non seulement à des fins cosmétiques mais aussi pour empêcher leur chute et pour les régénérer, a toujours sollicité l'esprit de recherche. De nombreuses théories ont tenté d'éclaircir l'étiologie de la chute des cheveux dans les cas de calvitie, d'alopécie, de pelade, etc., en les mettant sur le compte de la séborrhée, d'une augmentation de la tension du tissu sur la sphère crânienne, de l'irrigation réduite du sang, ou de certains troubles endocriniens ou nerveux.

Le follicule pileux est un mini-organe ancré dans la peau jusqu'à l'hypoderme, et dont la fonction principale est la production d'une tige pilaire. Leur distribution est établie au cours de la croissance *in utero* et leur nombre est déterminé génétiquement. Le follicule pileux est une structure dynamique qui produit le cheveu au cours de cycles de croissance et de remodelage de tissus. Ce cycle se décompose en 3 phases :
- Une phase de croissance (anagène), les cellules de la papille dermique (fibroblastes) envoient un signal aux cellules souches du bulbe qui permet leur prolifération. Ces cellules vont se transformer et envelopper la papille dermique pour former la matrice soufrée du cheveu. Elles se divisent et se différencient en kératinocytes, cellules responsables de la structure du cheveu. Pour que le cheveu soit bien structuré, les kératinocytes ont besoin de protéines soufrées, de vitamine B6 et de différents minéraux comme le zinc, le magnésium. La durée de cette phase détermine la longueur du cheveu et dépend de la prolifération et de la différenciation des cellules de la matrice à la base du follicule.

- Une phase de régression (catagène), la matrice meurt et de ce fait la papille dermique n'est plus en contact avec cette matrice. Il n'y a plus d'échange entre les cellules. Le follicule et la papille dermique remontent vers l'épiderme.
- Une phase de repos (télogène), les cellules de la papille dermique et du bulbe sont intactes et inactives. Le cheveu tombe. Pour qu'un nouveau cheveu se développe, il faut que le cycle soit réinitié.

Le développement et la croissance du follicule pileux sont influencés par des composés exprimés par la papille dermique, des protéines comme les Wnt et des facteurs de croissance comme ceux des kératinocytes (KGF) ou du facteur de croissance épithélial (Epithelium Growth Factor - EGF).

La chevelure se renouvelle donc en permanence et sur les 100 000 à 150 000 cheveux que comporte une chevelure, la majorité est en phase de croissance. Il existe une perte normale et physiologique de cheveux de l'ordre de 60 à 100 par jour pour une chevelure saine. Au-delà, la chute est dite pathologique qu'elle soit occasionnelle ou installée.

Le terme alopécie désigne la perte partielle ou générale des cheveux. De nombreux facteurs peuvent être impliqués dans l'alopécie comme par exemple les facteurs génétiques, l'âge, le sexe, les maladies, le stress, les problèmes hormonaux, les effets secondaires de médicaments, les cicatrices. Il est possible de distinguer plusieurs formes d'alopécie :
- L'alopécie androgénétique héréditaire, elle est la plus fréquente. La chute précoce des cheveux survient chez des sujets prédisposés génétiquement et elle atteint en particulier les hommes. Elle se manifeste par une diminution du volume des cheveux, voire une calvitie et touche 50% des hommes âgés de plus de 50 ans ;
- L'alopécie post-ménopausique, il s'agit de la plus fréquente cause de la calvitie chez la femme. Chez la femme, la chute des cheveux est plus diffuse et étendue que chez l'homme. L'alopécie diffuse féminine est un désordre qui démarre souvent à la ménopause et qui concerne environ 40% des femmes âgées de plus de 70 ans. Le terme diffus illustre que, contrairement à l'homme, la perte des cheveux concerne la totalité du cuir chevelu, de manière homogène ;
- L'alopécie aiguë ou réactionnelle, elle peut être liée à un traitement par chimiothérapie, un stress, un accouchement, des carences alimentaires importantes, une carence en fer, des troubles hormonaux, il s'agit d'une chute simultanée et diffuse d'une quantité importante de cheveux ;
- L'alopécie cicatricielle, elle peut être provoquée par des problèmes de peau (tumeur, brûlure, pelade), une irradiation aiguë, au lupus érythémateux ou des parasites (teigne, lichen) ;
- L'alopécie areata, elle semble être d'origine auto-immune et se caractérise par une atteinte en plaques plus ou moins larges et à un ou plusieurs endroits ;
- L'alopécie congénitale, rare, elle correspond à une absence de racines ou à des anomalies du cheveu (mutations).

L'alopécie est essentiellement liée à une perturbation du renouvellement capillaire qui entraine, dans un premier temps, l'accélération de la fréquence des cycles aux dépens de la qualité des cheveux puis de leur quantité. Le phénomène le plus fréquent est une réduction du cycle de croissance (phase anagène) dû à un arrêt de la prolifération cellulaire. La conséquence est une induction prématurée de la phase catagène et un nombre plus important de follicules pileux en phase télogène et donc une chute plus importante des cheveux. Pour lutter contre l'alopécie, il est donc nécessaire de relancer le cycle pilaire, par exemple, en activant la phase anagène.

A ce jour, il a été proposé différents produits pour lutter contre l'alopécie. La plupart associent plusieurs principes actifs susceptibles d'apporter une action bénéfique sur les paramètres biologiques impliqués dans la chute des cheveux. Parmi les principes actifs les plus couramment rencontrés, nous pouvons citer à titre d'exemples : des vitamines telles que les vitamines A, E, B5, B6, C, H et PP ; des oligo-éléments tels que le zinc, le cuivre, le magnésium, le silicium etc. ; des dérivés protéiques tels que les peptides, les acides aminés soufrés (type méthionine, cystine, cystéine ou dérivés) ; des huiles essentielles ou des extraits d'origine végétale de nature lipophile ou hydrophile dont la liste n'est pas limitative ; des agents antifongiques tels que la piroctonolamine, les dérivés undécycliniques, la cyclopiroxolamine... ; des molécules de synthèse chimique réputées pour leur action spécifique au niveau des récepteurs androgéniques ou sur l'activité des 5-α réductases. Le Minoxidil ou 2,4 diamino-6-pipéridinopyrimidine 3-oxide fait aujourd'hui référence dans le traitement de l'alopécie androgénique. En dépit des nombreuses théories évoquées sur son mécanisme d'action, ce dernier n'est pas clairement élucidé. De plus, son efficacité reste limitée, même s'il est constaté une stabilisation de la chute des cheveux dans de nombreux cas cliniques, en raison de la reprise du processus alopéciant dès l'arrêt du traitement. Son usage journalier contraignant est vraisemblablement à l'origine d'effets secondaires indésirables relevés chez des patients l'utilisant à long terme tels que réactions cutanées localisées ou effets systémiques.

FR2751541 décrit l'utilisation d'une composition comprenant un extrait de quinquina et un sel de zinc de l'acide carboxylique L-pyrrolidone pour limiter la chute des cheveux. FR2853245 décrit l'utilisation d'une composition comprenant un extrait de quinquina rouge pour traiter l'alopécie. EP2810697 décrit l'utilisation d'un extrait de Leontopodium alpinum pour stimuler la repousse capillaire. Il existe donc un besoin de nouveaux composés ou compositions utiles pour lutter contre l'alopécie.

### RESUME DE L'INVENTION

De façon surprenante, les inventeurs ont découvert que l'association d'extraits de quinquina et de *Leontopodium alpinum* et du sel de manganèse de l'acide carboxylique L-pyrrolidone, induisait une synergie d'action sur la voie Wnt-bCat, détaillée dans l'exemple 1.

L'invention concerne donc une association comprenant un extrait de quinquina, un extrait de *Leontopodium alpinum* et du sel de manganèse de l'acide carboxylique L-pyrrolidone.

### DEFINITIONS

Les termes « *Leontopodium alpinum* » et « edelweiss » sont interchangeables et utilisés indifféremment.

Par « extrait de *Leontopodium alpinum* », on entend désigner le produit d'extraction de tout ou partie de la plante edelweiss.

Par « extrait de quinquina », on entend désigner le produit d'extraction d'une ou plusieurs espèces de quinquinas, de préférence obtenus à partir des écorces de quinquinas.

Par « produit d'extraction », on entend le produit obtenu après extraction de tout ou partie de la plante à extraire avec un solvant, appelé solvant d'extraction, (c'est-à-dire une solution liquide dans le solvant d'extraction) éventuellement sous une forme concentrée ou sèche après évaporation partielle ou totale du solvant d'extraction.

Par « solvant polaire à moyennement polaire » on entend, au sens de la présente invention, un solvant ayant un moment dipolaire supérieure ou égale à 1,0 D. Il peut s'agir en particulier d'un solvant choisi dans le groupe constitué de l'eau, des alcools en C1 à C5 (par ex. éthanol), des glycols en C3 à C5 (par ex. propylène glycol, butylène glycol, pentylène glycol), du glycérol, de l'acétone, des esters d'alkyles en C1 à C5 (par ex. acétate d'éthyle, acétate d'isopropyle), des hydrocarbures halogénés en C1 à C5 (par ex. chloroforme, dichlorométhane), et des mélanges de ceux-ci.

Par « extrait hydro-alcoolique », on entend, au sens de la présente invention, un extrait obtenu à l'aide d'un solvant d'extraction consistant en un mélange alcool en C1 à C5 / eau, tel qu'un mélange éthanol/eau.

Par « extrait sec », on entend, au sens de la présente invention, un extrait dépourvu de solvant d'extraction ou de support, ou en contenant uniquement à l'état de trace non significative. Un tel extrait sec contient ainsi uniquement de la matière issue du végétal ayant été extrait.

Par « cheveux et/ou poils », on entend la chevelure, les poils, les sourcils, les cils et/ou le pelage, préférentiellement, les cheveux.

Par « alopécie » on entend la perte totale ou partielle des cheveux et/ou des poils, par exemple liée à la réduction de la croissance capillaire et/ou à l'accélération de la chute des cheveux et/ou des poils. Ce terme inclut mais ne se limite pas à l'alopécie androgénétique, l'alopécie post-ménopausique, l'alopécie réactionnelle, l'alopécie cicatricielle, l'alopécie areata, l'alopécie congénitale. Les conséquences de l'alopécie sont une absence temporelle ou définitive et partielle ou totale des cheveux et/ou des poils.

Par le terme « traiter » l'alopécie, on entend l'arrêt de l'alopécie, la réduction de l'alopécie et/ou l'atténuation de l'alopécie. Ainsi, « traiter » l'alopécie comprend limiter la chute des cheveux et/ou des poils, promouvoir la croissance des cheveux et/ou des poils, augmenter la densité des follicules pileux et/ou réguler les phases du cycle du follicule pileux.

Par le terme « prévenir » l'alopécie, on entend diminuer le risque d'apparition de l'alopécie, ou de ralentir la progression de l'alopécie chez un mammifère, préférentiellement l'homme qui est susceptible de développer une alopécie.

Par le terme « limiter », on entend freiner, réduire, diminuer et/ou stopper.

Par le terme « promouvoir », on entend augmenter, accroitre, favoriser, amplifier et/ou accélérer.

Dans la présente invention, on entend désigner par « cosmétiquement ou dermatologiquement acceptable » ce qui est utile dans la préparation d'une composition cosmétique ou dermatologique, qui est généralement sûre, non toxique et ni biologiquement ni autrement non souhaitable et qui est acceptable pour une utilisation cosmétique ou dermatologique, notamment par application topique et/ou par administration par voie orale.

### DESCRIPTION DETAILLEE

Selon un premier aspect, l'invention concerne une association comprenant un extrait de quinquina, un extrait de *Leontopodium alpinum* et du sel de manganèse de l'acide carboxylique L-pyrrolidone.

Les quinquinas sont connus depuis longtemps pour leurs propriétés médicinales. C'est le cas notamment du quinquina rouge (Cinchona succirubra), qui est un petit arbre pouvant atteindre 10 m de hauteur avec un tronc de 20 cm de diamètre, il conserve un feuillage vert toute l'année, il appartient à la famille des Rubiacées, originaire des régions équatoriales et plus particulièrement de l'Amérique du Sud et dont l'écorce est riche en quinine. Une fois prélevée, l'écorce tend à devenir brun rougeâtre sur sa face interne. Actuellement, des cultures existent en Asie du Sud-Est, en Amérique du Sud et en Afrique. L'écorce est importée d'Indonésie, de l'Inde, du Sri Lanka et partiellement d'Amérique du Sud et d'Afrique. L'écorce est obtenue par écorçage des racines, tronc et branches, l'écorce se régénère partiellement, il n'est pas utile de couper l'arbre.

Pour obtenir l'écorce, la première étape consiste en l'identification du Quinquina en fonction des besoins. Cette identification se fait grâce à la forme des feuilles. La sélection de l'arbre est réalisée en tenant compte de l'âge et donc de la grosseur des branches. Seules les branches ayant entre 6 et 8 ans sont sélectionnées. De plus, l'écorce doit être assez épaisse. Ainsi seules les écorces n'ayant jamais été récoltées ou s'étant régénérées sont sélectionnées. Après récolte, une écorce de quinquina se régénère en 2-3 ans en fonction des conditions climatiques. La récolte de l'écorce est manuelle à l'aide de machette. Elle se fait par temps sec de juillet à novembre. Afin de préserver la plante, seule l'écorce est maintenant récoltée sans couper le tronc. Les écorces récoltées sont conditionnées dans des sacs, puis acheminées vers le lieu de séchage. Celui-ci se déroule ainsi environ une vingtaine de jours après la récolte. Il consiste en un séchage naturel au soleil et dure environ 15 jours. Les écorces sont ensuite nettoyées pour éliminer les traces de mousses. Elles sont ensuite sélectionnées en fonction de leur taille. Un tamisage est réalisé pour éliminer les poussières.

L'extrait de quinquina de la présente invention est un extrait d'une ou plusieurs espèces de quinquina, et de préférence de l'écorce, choisies parmi la quarantaine d'espèces de quinquina connues à ce jour, entre lesquelles les hybridations sont nombreuses. De préférence, le quinquina selon l'invention est choisi parmi : les quinquinas gris (*Chinchona officinalis*), ils sont aromatiques, pauvres en tanin et en alcaloïdes ; les quinquinas jaunes (*Chinchina calisaya*) qui sont les quinquinas les plus riches en alcaloïdes totaux et en quinine ; les quinquinas rouges (*Chinchona succirubra*) qui sont riches en tanin, et plus riches que les quinquinas gris en alcaloïdes ; les quinquinas à quinine ; et des associations de ceux-ci. De façon préférée, le quinquina selon l'invention est choisi parmi les quinquinas rouges.

Des extraits d'écorces de quinquina sont connus pour certaines propriétés thérapeutiques. Ainsi, les écorces de quinquina sont astringentes par leur tanin, toniques amères, fébrifuges et anti-malariques par leurs alcaloïdes, notamment la quinine. Les propriétés antipyrétiques de l'écorce sont dues principalement à la quinine, et dans une moindre mesure aux alcaloïdes quinidine, cinchonine, cinchonidine. Le quinquina est tonique grâce à son acide quinotannique qui est combiné en partie avec des alcaloïdes. L'action tonique est aussi due à la quinovine.

Dans la présente invention, l'extrait de quinquina est utilisé à titre de principe actif et non pas de tonique.

D'une façon préférée, l'extrait de quinquina selon l'invention est un extrait éthanolique d'écorce de quinquina, notamment de quinquina rouge.

L'extrait de quinquina selon l'invention contient avantageusement 1 à 10% en poids de quinine par rapport au poids de l'extrait sec. En outre, l'extrait de quinquina selon l'invention contient avantageusement entre 4 et 20% en poids d'alcaloïdes totaux (dont la quinine) par rapport au poids de l'extrait sec. L'extrait de quinquina selon l'invention pourra comprendre également entre 5 et 10% en poids de proanthocyanes (polyphénols) exprimés en procyanidine B2, par rapport au poids de l'extrait sec.

Par exemple, l'extrait selon la présente invention peut être obtenu par extraction à l'éthanol d'écorces séchées et broyées de quinquina, notamment de quinquina rouge, le mélange étant ensuite filtré pour récupérer l'extrait. Lors de l'extraction, le rapport volumique plante/éthanol est par exemple compris entre 1/5 et 1/10, notamment entre 1/7 et 1/9. Après filtration, l'extrait peut être stabilisé par l'addition d'acide citrique (par ex.0,1 à 1% (p/v) tel que 0,4% (p/v)). L'extrait ainsi obtenu peut être un liquide brun-rouge contenant de 3 à 5% de matière sèche. L'extrait ainsi obtenu contient avantageusement 0,06 à 0,3% (p/v) de quinine. L'extrait contient en outre entre 0,2 et 0,6% (p/v) d'alcaloïdes totaux (dont la quinine) et environ 0,3% (p/v) de proanthocyanes (polyphénols) exprimés en procyanidine B2. L'extrait obtenu peut être utilisé en l'état ou sous forme d'extrait concentré ou d'extrait sec après évaporation partielle ou totale du solvant d'extraction (éthanol).

L'edelweiss (*Leontopodium alpinum* encore appelé *Leontopodium nivale*) est une espèce de plante de la famille des Astéracées. C'est une des plus célèbres plantes de montagne, en partie en raison de sa rareté dans son environnement naturel, elle pousse à une altitude de 1500 à 3000 mètres, dans des zones peu hospitalières (ravines, rocheuses, froides et très exposées aux UV). Cette plante s'est parfaitement adaptée à ces conditions extrêmes au travers d'une panoplie de molécules d'intérêts et grâce à des poils protecteurs sur sa fleur et ses feuilles. En effet, ses feuilles sont feutrées de poils blancs laineux, ses fleurs sont également feutrées de poils blancs laineux avec une inflorescence caractéristique en assemblage de 5 à 6 petits capitules jaunes entourés de folioles disposées en étoile. Cette plante est trouvée dans les Alpes, mais aussi dans les Pyrénées, les Carpates et les Balkans. L'edelweiss est partiellement protégé, mais cette plante est également cultivée. Depuis peu, l'homme maîtrise la culture des cellules issues de toutes petites parties des végétaux comme un fragment de feuille, de racine, de tige. Cet outil ouvre la voie à la production de biomasse et de molécules d'intérêts.

La plante n'est pas toxique, bien que non comestible, des décoctions de fleurs dans du lait sont encore régulièrement pratiquées. Cette plante est utilisée en médecine populaire contre les douleurs abdominales, les angines, les bronchites et les diarrhées ou dysenteries. L'industrie cosmétique s'intéresse à ses vertus antioxydantes. Des extraits de racines séchées de *Leontopodium alpinum* possèdent des propriétés anti-inflammatoires. La grande majorité des propriétés de cette plante est attribuée à la présence de métabolites secondaires polyphénoliques. L'edelweiss contient une large variété de polyphénols appartenant aux classes des phénylpropanoïdes (acide phénolique, glycosides, flavonoïdes, coumarines et lignanes), des terpènes (sesquiterpènes et acides diterpéniques) et des alcaloïdes (dérivés benzofuranes et pyranes). L'évaluation analytique de parties aériennes de l'edelweiss révèle des glycosides et des aglycones de flavonoïdes (lutéoline, quercétine, et apigénine) aussi bien que des acides leontopodique, chlorogénique, et 3,5-dicaffeoylquinique.

Dans le cadre de la présente invention, l'extrait *Leontopodium alpinum* ou extrait d'edelweiss peut être obtenu à partir de tout ou partie de la plante (edelweiss) choisie parmi les parties aériennes telles que les feuilles, tiges, fleurs, graines ; les parties souterraines telles que les racines ; et des combinaisons de celles-ci. Avantageusement il s'agit de parties aériennes.

La plante ou partie de plante edelweiss peut être fraiche ou sèche, entière, coupée ou broyée, puis soumise à une étape d'extraction.

Un procédé de préparation d'un extrait selon l'invention comprend avantageusement une étape d'extraction de tout ou partie de la plante *Leontopodium alpinum* par un solvant d'extraction avantageusement choisi parmi les solvants polaires à moyennement polaires.

Le solvant d'extraction sera de préférence choisi dans le groupe constitué de l'eau, des alcools en C1 à C5 (par ex. éthanol), des glycols en C3 à C5 (par ex. propylène glycol, butylène glycol, pentylène glycol), du glycérol, de l'acétone, des esters d'alkyles en C1 à C5 (par ex. acétate d'éthyle, acétate d'isopropyle), des hydrocarbures halogénés, notamment chlorés, en C1 à C5 (par ex. chloroforme, dichlorométhane), des mélanges de ceux-ci.

Dans un mode de réalisation de l'invention, le solvant d'extraction est un ester d'alkyles en C1 à C5 (par ex. l'acétate d'éthyle, l'acétate d'isopropyle), un alcool en C1 à C5 (par ex. l'éthanol), un mélange alcool en C1 à C5/eau (par ex. éthanol/eau) ou de l'eau. Avantageusement, il s'agira d'un mélange éthanol/eau, utilisé par exemple dans un rapport volumique 1/10 à 10/1.

Selon un mode de réalisation particulier de l'invention, l'extraction est réalisée sous agitation ou de façon statique, à reflux, à température ambiante, ou à une température comprise entre la température ambiante et le reflux. Elle peut être assistée par ultra-sons, par micro-ondes, par flash détente ou par extrusion. L'extraction peut être réalisée dans un ratio poids de plantes / volume de solvant d'extraction pouvant varier de 1/3 à 1/30, notamment pendant une durée de 1 minute à 48 heures, par exemple de 10 minutes à 24 heures. L'extraction peut être renouvelée 2 à 3 fois. Le marc est ensuite séparé de l'extrait, notamment par centrifugation ou filtration, et la solution peut éventuellement être plus ou moins concentrée pouvant aller jusqu'à l'obtention d'un extrait sec. Un support peut être rajouté lors de l'étape de concentration de façon à obtenir un extrait contenant 1 à 75% d'extrait sec. Le support peut être de la maltodextrine, du lactose, de la silice, de la glycérine, un glycol (par ex. 1,2-pentanediol, 1,3-butanediol, 1,3-propanediol), une huile végétale, ou tout autre support cosmétologiquement acceptable et solubilisant l'extrait, préférentiellement d'origine biosourcée, ou un mélange de ceux-ci. L'extrait peut également être décoloré, par exemple sur charbon actif, de façon à éliminer tout ou partie des chlorophylles.

Selon un mode préféré de l'invention, l'extrait de *Leontopodium alpinum* est préparé comme suit : les parties aériennes de *Leontopodium alpinum* sont séchées sous un flux d'air chaud et ensuite broyées. Puis une extraction est réalisée avec une solution éthanol/eau, utilisé par exemple dans un rapport volumique 1/10 à 10/1. L'éthanol est ensuite enlevé par une distillation sous vide. Le concentré est formulé avec de la glycérine comme support et filtré.

Les extraits de *Leontopodium alpinum* selon l'invention peuvent également être obtenus grâce à la technologie de culture de cellules végétales dirigée. Par exposition des cultures à de fines variations physiques ou nutritionnelles par exemple, il est possible de moduler leurs processus métaboliques et favoriser ainsi l'augmentation de la synthèse de molécules d'intérêts, comme par exemple l'acide leontopodique.

Selon un mode de réalisation particulier de l'invention, l'extrait de *Leontopodium alpinum* selon l'invention est caractérisé par une teneur de 0,05 à 1 % en acide leontopidique, % en poids par rapport au poids de l'extrait sec, de préférence d'au moins 0,1% en acide leontopidique, % en poids par rapport au poids de l'extrait sec.

Le manganèse fait partie des oligo-éléments essentiels au bon équilibre de l'organisme. Il est utile aux cellules à très faibles doses, le manganèse contribue à l'activité de nombreuses enzymes et notamment à celle de la superoxyde dismutase. L'organisme dispose de plusieurs superoxyde dismutases métallo-dépendantes mais celles activées par le manganèse sont essentielles pour la protection de la peau. En effet, situées dans les mitochondries, elles constituent la première ligne de défense de l'organisme contre les radicaux superoxydes produits à la suite de réactions d'inflammation ou d'une exposition de la peau aux UV. L'induction de la superoxyde dismutase manganèse-dépendante peut être régulée par les anions superoxydes eux-mêmes mais aussi par différentes cytokines comme l'IL-1α et le TNFα. La réaction de dismutation des anions superoxydes en peroxyde d'hydrogène est spontanée mais la superoxyde dismutase manganèse-dépendante en accélère la vitesse et les radicaux superoxydes sont éliminés 10¹⁰ fois plus vite.

Le manganèse est nécessaire au bon fonctionnement du cerveau, le manganèse est efficace pour le traitement de nombreux troubles nerveux. Indispensable au métabolisme et à la production de l'énergie (co-facteur de la pyruvate kinase retrouvée dans le cycle de Krebs, de l'arginase, enzyme transformant l'arginine en urée), il possède également un rôle majeur dans la fabrication des protéines et des acides nucléiques.

L'acide carboxylique L-pyrrolidone est considéré comme un agent hydratant lorsqu'il se trouve associé à différents cations (sodium, potassium, calcium...), il entre à 12% dans la composition du facteur naturel d'hydratation cutané, partie aqueuse du film hydrolipidique de surface de l'épiderme. L'acide carboxylique L-pyrrolidone occupe une place centrale dans la biochimie de l'organisme et de la peau, il est le trait d'union entre le métabolisme énergétique, le pool protéique et l'hydratation de la peau. D'un point de vue biologique, l'acide carboxylique L-pyrrolidone est considéré comme un transporteur naturel qui véhicule au cœur des cellules les cations qui lui sont associés.
Ainsi, le sel de manganèse de l'acide carboxylique L-pyrrolidone constitue un apport physiologique en manganèse biodisponible afin de stimuler l'activation de la superoxyde dismutase manganèse-dépendante et de renforcer les défenses naturelles anti-radicalaires de la peau selon un double effet, préventif et curatif. Le sel de manganèse de l'acide carboxylique L-pyrrolidone dispose d'une efficacité lui permettant de stimuler, en l'absence d'agression, la concentration basale de la peau en superoxyde dismutase manganèse-dépendante et donc renforce son niveau de défense anti-radicalaire. Par ailleurs il permet de combattre efficacement les radicaux libres en augmentant la réponse de la peau exposée aux radiations UV.

Dans un mode de réalisation particulier, l'invention vise une association comprenant un extrait de quinquina rouge, en particulier un extrait d'écorce de quinquina rouge, un extrait de *Leontopodium alpinum* et du sel de manganèse de l'acide carboxylique L-pyrrolidone.

Selon un autre mode particulier, l'invention vise une association comprenant un extrait de quinquina rouge, en particulier un extrait d'écorce de quinquina rouge, un extrait hydro-alcoolique de *Leontopodium alpinum* et du sel de manganèse de l'acide carboxylique L-pyrrolidone.

Selon un deuxième aspect, la présente invention concerne aussi une composition cosmétique ou dermatologique comprenant une association selon l'invention selon un mode décrit précédemment et au moins un excipient cosmétiquement ou dermatologiquement acceptable, plus particulièrement adaptée pour une application topique et/ou pour une administration par voie orale, de préférence pour une application topique.

Dans un mode de réalisation particulier, l'association selon l'invention est le seul principe actif anti-alopécie de la composition.

Dans un autre mode de réalisation particulier, les compositions cosmétiques ou dermatologiques selon l'invention comprennent au moins un autre principe actif anti-alopécie tel que le finastéride ou le minoxidil.

L'invention vise de préférence des compositions cosmétiques ou dermatologiques selon l'invention se présentant sous une forme propre et adaptée à une application topique.

Les compositions cosmétiques ou dermatologiques selon l'invention pourront se présenter ainsi sous les formes qui sont habituellement connues pour une administration topique, c'est-à-dire notamment les lotions, les shampoings, les baumes, les mousses, les gels, les dispersions, les émulsions, les sprays, les sérums, les masques ou les crèmes, avec des excipients permettant notamment une pénétration afin d'améliorer les propriétés et l'accessibilité des principes actifs.

Avantageusement, les compositions selon l'invention peuvent se présenter sous les formes qui sont habituellement connues pour une administration topique sur les cheveux et le cuir chevelu, c'est-à-dire notamment un shampoing, un après-shampoing, une crème capillaire, une lotion capillaire, un masque ou un spray sans rinçage.

On distingue ainsi des produits formulés pouvant être rincés et des produits formulés ne nécessitant pas de rinçage.

Ces compositions contiennent généralement, outre les composés et extraits de l'association selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents hydratants ou des eaux thermales, etc.

Avantageusement, les compositions selon la présente invention comprendront 0,01% à 10% en poids, de préférence 0,1 à 8% en poids, de manière préférée 0,5% à 6% en poids, de manière encore préférée 1% à 5% en poids d'extrait de quinquina, par rapport au poids total de la composition. D'une manière préférée, la composition comprendra 3,0% en poids d'extrait de quinquina, par rapport au poids total de la composition. D'une manière toute aussi préférée, la composition comprendra 5,0% en poids d'extrait de quinquina, par rapport au poids total de la composition. Les extraits de quinquina utilisés dans les compositions selon l'invention comprendront avantageusement une teneur d'extrait sec de 1% à 10%, de préférence de 1% à 5%, en poids par rapport au poids total de l'extrait.

Selon un autre mode de réalisation, les compositions selon la présente invention comprendront 0,0001% à 1%, de préférence 0,001 à 0,8% en poids, de manière préférée 0,005% à 0,6% en poids, de manière encore préférée 0,01% à 0,5% d'extrait de quinquina, en poids d'extrait sec par rapport au poids total de la composition. Les extraits de quinquina utilisés dans les compositions selon l'invention comprendront avantageusement une teneur d'extrait sec de 1% à 10%, de préférence de 1% à 5%, en poids par rapport au poids total de l'extrait.

Avantageusement, les compositions selon la présente invention comprendront 0,01% à 5% en poids, de préférence 0,05% à 2% en poids, de manière préférée 0,1% à 1,0% en poids d'extrait de *Leontopodium alpinum*, par rapport au poids total de la composition. D'une manière préférée, la composition comprendra 0,5% en poids d'extrait de *Leontopodium alpinum,* par rapport au poids total de la composition. D'une manière toute aussi préférée, la composition comprendra 1,0% en poids d'extrait de *Leontopodium alpinum*, par rapport au poids total de la composition. Les extraits de *Leontopodium alpinum* utilisés dans les compositions selon l'invention comprendront avantageusement une teneur d'extrait sec de 1% à 20%, de préférence de 5% à 10%, en poids par rapport au poids total de l'extrait.

Selon un autre mode de réalisation, les compositions selon la présente invention comprendront 0,0001% à 1%, de préférence 0,0005 à 0,5% en poids, de manière préférée 0,005% à 0,6% en poids, de manière encore préférée 0,001% à 0,2%, de manière encore plus préférée 0,005% à 0,1% d'extrait de *Leontopodium alpinum*, en poids d'extrait sec par rapport au poids total de la composition. Les extraits de *Leontopodium alpinum* utilisés dans les compositions selon l'invention comprendront avantageusement une teneur d'extrait sec de 1% à 20%, de préférence de 5% à 10%, en poids par rapport au poids total de l'extrait.

Avantageusement, les compositions selon la présente invention comprendront 0,01% à 1,5% en poids, de préférence 0,01 à 1% en poids, de manière préférée 0,05% à 0,5% en poids de sel de manganèse de l'acide carboxylique L-pyrrolidone, par rapport au poids total de la composition. D'une manière préférée, la composition comprendra 0,1% en poids de sel de manganèse de l'acide carboxylique L-pyrrolidone, par rapport au poids total de la composition. D'une manière toute aussi préférée, la composition comprendra 0,5% en poids de sel de manganèse de l'acide carboxylique L-pyrrolidone, par rapport au poids total de la composition.

Selon un mode de réalisation préféré, les compositions selon la présente invention comprennent :
- 0,01% à 10% en poids, de préférence 0,1% à 8% en poids, de manière préférée 0,5% à 6% en poids, de manière encore préférée 1 à 5% en poids d'extrait de quinquina, par rapport au poids total de la composition, l'extrait de quinquina comprenant avantageusement une teneur d'extrait sec de 1% à 10%, de préférence de 1% à 5%, en poids par rapport au poids total de l'extrait ;
- 0,01% à 5% en poids, de préférence 0,05% à 2% en poids, de manière préférée 0,1° à 1% en poids d'extrait de *Leontopodium alpinum,* par rapport au poids total de la composition, l'extrait de *Leontopodium alpinum* comprenant avantageusement une teneur d'extrait sec de 1% à 20%, de préférence de 5% à 10%, en poids par rapport au poids total de l'extrait ;
- 0,01% à 1,5% en poids, de préférence 0,01% à 1% en poids, de manière préférée 0,05% à 0,5% en poids, de manière encore préférée 0,05% à 0,2% en poids de sel de manganèse de l'acide carboxylique L-pyrrolidone, par rapport au poids total de la composition.

Selon un autre mode de réalisation préféré, les compositions selon la présente invention comprennent :
- 0,0001% à 1%, de préférence 0,001 à 0,8% en poids, de manière préférée 0,005% à 0,6% en poids, de manière encore préférée 0,01% à 0,5% d'extrait de quinquina, en poids d'extrait sec par rapport au poids total de la composition ;
- 0,0001% à 1%, de préférence 0,0005 à 0,5% en poids, de manière préférée 0,005% à 0,6% en poids, de manière encore préférée 0,001% à 0,2%, de manière encore plus préférée 0,005% à 0,1% d'extrait de *Leontopodium alpinum,* en poids d'extrait sec par rapport au poids total de la composition ;
- 0,01% à 1,5% en poids, de préférence 0,01% à 1% en poids, de manière préférée 0,05% à 0,5% en poids, de manière encore préférée 0,05% à 0,2% en poids de sel de manganèse de l'acide carboxylique L-pyrrolidone, par rapport au poids total de la composition.

De manière particulière, lorsque l'on exprime les quantités d'extrait de quinquina, d'extrait de *Leontopodium alpinum* et de sel de manganèse de l'acide carboxylique L-pyrrolidone dans l'association ternaire selon l'invention, en parts, cette association comprend entre 10 et 100 parts d'extrait de quinquina, entre 1 et 10 parts d'extrait de *Leontopodium alpinum* et entre 0,5 et 5 parts de sel de manganèse de l'acide carboxylique L-pyrrolidone. De manière préférée, l'association selon l'invention comprend, en parts, 30 ou 50 parts d'extrait de quinquina, 5 parts d'extrait de *Leontopodium alpinum* et 1 part de sel de manganèse de l'acide carboxylique L-pyrrolidone.

De préférence, la composition selon l'invention a une texture légère permettant en outre une pénétration optimale sans graisser les cheveux et/ou les poils. Dès les premières applications, la chevelure retrouvera force et vitalité.

Les compositions selon l'invention peuvent être fabriquées selon des procédés bien connus de l'homme du métier.

Selon un troisième aspect, l'invention vise une association selon l'invention selon un mode décrit précédemment ou une composition cosmétique ou dermatologique selon l'invention selon un mode décrit précédemment, pour son utilisation dans la prévention et/ou le traitement de l'alopécie.

L'invention concerne également l'utilisation d'une association selon l'invention selon un mode décrit précédemment ou une composition cosmétique ou dermatologique selon l'invention selon un mode décrit précédemment, pour la fabrication d'une composition dermatologique destinée à la prévention et/ou au traitement de l'alopécie.

L'invention concerne également une méthode de prévention et/ou traitement de l'alopécie comprenant l'administration à un individu en ayant besoin d'une quantité efficace d'une association selon l'invention selon un mode décrit précédemment ou d'une composition cosmétique ou dermatologique selon l'invention selon un mode décrit précédemment.

L'alopécie peut être choisie dans le groupe constitué par l'alopécie androgénétique, l'alopécie post-ménopausique, l'alopécie réactionnelle et l'alopécie areata. Plus particulièrement, l'alopécie est choisie dans le groupe constitué par l'alopécie androgénétique et l'alopécie réactionnelle. De préférence, l'alopécie est l'alopécie androgénétique.

Dans le cadre de la prévention et/ou du traitement de l'alopécie, l'association selon l'invention ou la composition cosmétique ou dermatologique selon l'invention sera avantageusement administrée par voie topique et/ou par voie orale.

L'association selon l'invention ou la composition cosmétique ou dermatologique selon l'invention peut être utilisée en combinaison avec un traitement de l'alopécie, comme le finastéride ou le minoxidil, et/ou en combinaison avec des composés utiles pour une bonne structure du cheveu, comme par exemple des protéines soufrées, de la vitamine B6 et différents minéraux comme le zinc et/ou le magnésium.

L'association selon l'invention ou la composition cosmétique ou dermatologique selon l'invention peut être utilisée chez un individu qui a subi ou va subir une micro greffe.

Selon un quatrième aspect, l'invention vise une utilisation cosmétique de l'association selon l'invention selon un mode décrit précédemment ou de la composition cosmétique ou dermatologique selon l'invention selon un mode décrit précédemment, pour limiter la chute des cheveux et/ou des poils et/ou pour promouvoir la croissance capillaire et/ou pour augmenter la densité des follicules pileux et/ou pour obtenir une chevelure plus couvrante et/ou pour favoriser la régénération folliculaire.

L'invention concerne également une méthode cosmétique pour limiter la chute des cheveux et/ou des poils et/ou pour promouvoir la croissance capillaire et/ou pour augmenter la densité des follicules pileux et/ou pour obtenir une chevelure plus couvrante et/ou pour favoriser la régénération folliculaire comprenant l'administration à un individu en ayant besoin d'une quantité efficace d'une association selon l'invention selon un mode décrit précédemment ou d'une composition cosmétique ou dermatologique selon l'invention selon un mode décrit précédemment.

L'association selon l'invention ou la composition cosmétique ou dermatologique selon l'invention sera avantageusement administrée par voie topique et/ou par voie orale.

L'association selon l'invention ou la composition cosmétique ou dermatologique selon l'invention peut être utilisée en combinaison avec des composés utiles pour limiter la chute des cheveux et/ou des poils et/ou pour promouvoir la croissance capillaire et/ou pour augmenter la densité et/ou pour obtenir une chevelure plus couvrante et/ou pour favoriser la régénération folliculaire et/ou en combinaison avec des composés utiles pour une bonne structure du cheveu, comme par exemple des protéines soufrées, de la vitamine B6 et différents minéraux comme le zinc et/ou le magnésium.

L'association selon l'invention ou la composition cosmétique ou dermatologique selon l'invention permet ainsi d'enrayer la chute du cheveu, d'en prolonger son cycle, de sorte que le capital cheveu est préservé, en quantité et en qualité.

Selon un cinquième aspect, la présente invention concerne également un procédé cosmétique pour limiter la chute des cheveux et/ou des poils et/ou promouvoir la croissance capillaire et/ou augmenter la densité des follicules pileux et/ou pour obtenir une chevelure plus couvrante et/ou pour favoriser la régénération folliculaire comprenant une étape d'administration de l'association selon l'invention selon un mode décrit précédemment ou la composition cosmétique selon l'invention selon un mode décrit précédemment à un individu.

Selon un mode de réalisation particulier, il s'agit d'un procédé de soin cosmétique des cheveux destiné à améliorer l'esthétique des cheveux en favorisant la pousse capillaire et/ou à obtenir une chevelure plus couvrante caractérisé en ce qu'il consiste à appliquer sur les cheveux et le cuir chevelu une quantité efficace d'une association selon l'invention selon un mode décrit précédemment ou d'une composition selon l'invention selon un mode décrit précédemment, à laisser celle-ci au contact des cheveux et du cuir chevelu, et éventuellement à rincer les cheveux et le cuir chevelu pour éliminer la dite association ou composition.

Les exemples qui suivent illustrent l'invention sans en limiter la portée.

### Exemple 1 : test pharmacologique d'un extrait de quinquina, de sel de manganèse de l'acide carboxylique L-pyrrolidone et d'un extrait de Leontopodium alpinum et de leur association

Le but de cette étude est d'évaluer les effets de l'association d'un extrait de quinquina, de sel de manganèse de l'acide carboxylique L-pyrrolidone et d'un extrait de *Leontopodium alpinum* sur l'activation de la voie Wnt/β-caténine sur des cellules de la papille dermique issue de follicules pileux humains.

Le développement et la croissance du follicule pileux sont influencés par des composés exprimés par la papille dermique : des protéines comme les Wnt et des facteurs de croissance comme ceux des kératinocytes (KGF). Ils sont impliqués dans des voies de communication intercellulaires et sont connus pour agir sur les kératinocytes du follicule.

Lors de la transition de la phase de repos à la phase de croissance, les cellules souches du bulge sont activées par le signal Wnt qui régule l'expression de leurs gènes. Une augmentation de β-caténine intracellulaire est détectée dans la base du bulge. La régénération du cheveu commence.

Wnt est une famille de glycoprotéines dont le nom correspond à la réunion de Wg (wingless) et Int (intégration site). La voie de signalisation des protéines Wnt via la β-caténine est appelée canonique, c'est-à-dire la voie privilégiée. Le signal Wnt active la régénération du cheveu et participe à sa croissance. Wnt se fixe sur les récepteurs extracellulaires permettant la stabilisation de la β-caténine intracellulaire, empêchant ainsi sa dégradation par le protéasome. La β-caténine peut alors pénétrer le noyau et jouer un rôle de co-activateur de facteurs de transcription et stimuler l'expression de gènes spécifiques impliqués dans la croissance du cheveu comme le gène codant pour les KGF.

Le développement du cheveu dépend d'une boucle de signalisation entre les kératinocytes et les cellules de la papille dermique. L'expression de Wnt au niveau des kératinocytes induit l'augmentation de β-caténine dans les cellules de la papille dermique régulant des voies de signalisation incluant les facteurs de croissance qui guident la morphogenèse du cheveu. KGF est un médiateur paracrine endogène important dans le développement, la différenciation et la croissance du follicule pileux. β-caténine et KGF sont fortement présents en phase anagène puis disparaissent. Une perte d'expression des β-caténines stoppe le signal Wnt et induit le passage en phase catagène.

### Protocole expérimental

Les études sont réalisées sur des cellules humaines issues des papilles dermiques des follicules de trois donneurs. Les cellules sont ensemencées dans des plaques de 96 puits et mises en culture pendant 24 h avec les compléments nécessaires. Les cellules sont incubées avec des réactifs spécifiques afin d'être transfectées avec un lentivirus (exprimant le gène de la luciférase sous le contrôle du promoteur Wnt/β-caténine (TCF/LEF transcriptional response element). Les cellules transfectées sont incubées pendant 24 heures avec les produits à tester, c'est-à-dire soit avec un extrait de quinquina (10 µg/ml) dilué dans du DMSO, soit avec le sel de manganèse de l'acide carboxylique L-pyrrolidone (500µM) dilué dans l'eau, soit avec un extrait de *Leontopodium alpinum* (30µg/ml) dilué également dans l'eau ou bien encore avec l'association des trois composés aux mêmes concentrations que précédemment. Le quinquina utilisé dans cette étude est récolté en Equateur, dans les forêts d'Echéandia. L'extrait de *Leontopodium alpinum* testé dans cette étude provient d'une variété appelée *Leontopodium alpinum Helvetia.* L'extrait testé correspond à la matière commerciale Alpaflor^{®} du fournisseur DSM. Le sel de manganèse de l'acide carboxylique L-Pyrrolidone testé dans cette étude correspond à la matière commerciale Mangalidone^{®} du fournisseur Solabia. Il est obtenu par cyclisation de l'acide glutamique d'origine végétale. Son nom INCI est le manganèse PCA et son numéro CAS est le 29193-02-2.

Un contrôle positif (la protéine Wnt3a à 10nM) est également testé. l'activation de la voie Wnt/β-caténine est mise en avant par la quantification de la luminescence due à l'expression de la luciférase.

Une analyse statistique est réalisée, une comparaison intergroupe est faite par une ANOVA à mesures répétées suivie par un post test de Dunnett sur les données brutes.

Le tableau 1 suivant représente l'activation de la voie Wnt/β-caténine après une incubation des différents composés seuls ou associés.

**Tableau 1**

| Traitement | | Moyenne de 3 donneurs (6 expériences) | | |
|---|---|---|---|---|
| Composés testés | Conc | Moyenne RLU | e.s.m. | Stats vs contrôle |
| Contrôle | | 410,2 | 38,5 | - |
| Wnt3a | 10nM | 2366,9 | 743,6 | P<0,01 |
| Quinquina (Q) | 10µg/mL | 412,2 | 40,8 | P=NS |
| Mn-PCA (L) | 500µM | 735,2 | 135,5 | P<0,05 |
| Edelweiss (E) | 30µg/mL | 722,1 | 104,8 | P<0,05 |
| Q + L + E | 10+500+30 | 1030,4 | 176,7 | P<0,01 |

Conc : concentrations ; e.s.m. : erreur standard à la moyenne ; stats : analyses statistiques ; Q : extrait de quinquina ; Mn-PCA : sel de manganèse de l'acide carboxylique L-pyrrolidone ; E : extrait d'Edelweiss, c'est-à-dire de *Leontopodium alpinum* ; Q+L+E : extrait de quinquina + sel de manganèse de l'acide carboxylique L-pyrrolidone + extrait d'Edelweiss.

Le traitement des cellules humaines issues des papilles dermiques de follicules avec le contrôle positif des conditions expérimentales, Wnt3a à 10nM, induit une forte activation statistiquement significative de la voie Wnt-β-caténine (+451 ± 93%, p<0,01 versus contrôle). Ce résultat attendu permet de valider le test et les conditions expérimentales.

Le traitement des cellules humaines issues des papilles dermiques de follicules avec l'extrait de quinquina à 10µg/mL ne montre aucune modulation significative de la voie Wnt-β-caténine.

Le traitement des cellules humaines issues des papilles dermiques de follicules avec le sel de manganèse de l'acide carboxylique L-pyrrolidone à 500µM induit une activation significative de la voie Wnt-β-caténine (+77 ± 17%, p<0,05 versus contrôle).

De même, dans les mêmes conditions, l'extrait d'Edelweiss testé à 30µg/mL active significativement la voie Wnt-β-caténine (+75 ± 8%, p<0,05 versus contrôle).

Le traitement des cellules humaines issues des papilles dermiques de follicules avec l'association de l'extrait de quinquina à 10µg/mL, du sel de manganèse de l'acide carboxylique L-pyrrolidone à 500µM et de l'extrait d'Edelweiss à 30µg/mL induit une forte activation statistiquement significative de la voie Wnt-β-caténine (+148 ± 19%, p<0,01 versus contrôle). Le tableau 2 ci-dessous résume l'analyse statistique réalisée entre les différents groupes.

**Tableau 2**

| Traitement | | Analyse statistique | |
|---|---|---|---|
| Composés testés | Conc | Versus contrôle | Versus Q + L + E |
| Contrôle | - | - | P<0,01 |
| Quinquina (Q) | 10µg/mL | P=NS | P<0,01 |
| Mn-PCA (L) | 500µM | P<0,05 | P<0,01 |
| Edelweiss (E) | 30µg/mL | P<0,05 | P<0,05 |
| Q + L + E | 10+500+30 | P<0,01 | - |

Les inventeurs mettent ainsi en évidence que cette association (extrait de quinquina, extrait de *Leontopodium alpinum* et sel de manganèse de l'acide carboxylique L-pyrrolidone) induit une activation plus importante de la voie Wnt-β-caténine, que celui des trois composés pris isolément. Les inventeurs démontrent ainsi une synergie d'action en associant ces trois composés.

## Revendications

1. Association comprenant un extrait de quinquina, un extrait de *Leontopodium alpinum,* et du sel de manganèse de l'acide carboxylique L-pyrrolidone.

2. Association selon la revendication 1, **caractérisée en ce que** l'extrait de quinquina est un extrait d'écorce de quinquina rouge.

3. Association selon l'une quelconque des revendications 1 et 2, **caractérisée en ce que** l'extrait de *Leontopodium alpinum* est un extrait hydro-alcoolique.

4. Association selon l'une quelconque des revendications 1 à 3, pour son utilisation dans la prévention et/ou le traitement de l'alopécie.

5. Association pour son utilisation selon la revendication 4 dans laquelle l'alopécie est choisie dans le groupe constitué par l'alopécie androgénétique, l'alopécie réactionnelle, l'alopécie post-ménopausique et l'alopécie areata.

6. Utilisation non-thérapeutique de l'association selon l'une quelconque des revendications 1 à 3, pour limiter la chute des cheveux et/ou des poils et/ou pour promouvoir la croissance capillaire et/ou pour augmenter la densité des follicules pileux et/ou pour obtenir une chevelure plus couvrante et/ou pour favoriser la régénération folliculaire.

7. Composition cosmétique ou dermatologique comprenant à titre de principe actif une association selon l'une quelconque des revendications 1 à 3, avec au moins un excipient cosmétiquement ou dermatologiquement acceptable.

8. Composition selon la revendication 7, **caractérisée en ce qu'**elle comprend 0,01% à 10%, notamment 0,5% à 6%, de préférence 1% à 5%, en poids d'un extrait de quinquina par rapport au poids total de la composition.

9. Composition selon l'une quelconque des revendications 7 et 8, **caractérisée en ce qu'**elle comprend 0,01% à 1,5%, notamment 0,01% à 1,0%, de préférence 0,05% à 0,5%, en poids du sel de manganèse de l'acide carboxylique L-pyrrolidone par rapport au poids total de la composition.

10. Composition selon l'une quelconque des revendications 7 à 9, **caractérisée en ce qu'**elle comprend 0,01% à 5%, notamment 0,05% à 2%, de préférence 0,1% à 1%, en poids d'un extrait de *Leontopodium alpinum* par rapport au poids total de la composition.

11. Composition selon l'une quelconque des revendications 7 à 10, **caractérisée en ce qu'**elle comprend en outre un autre principe actif anti-alopécie.

12. Composition selon l'une quelconque des revendications 7 à 11, **caractérisée en ce qu'**elle se présente sous une forme propre à une application topique.

13. Utilisation non-thérapeutique d'une composition cosmétique selon l'une quelconque des revendications 7 à 12 pour limiter la chute des cheveux et/ou des poils et/ou pour promouvoir la croissance capillaire et/ou pour augmenter la densité des follicules pileux et/ou pour obtenir une chevelure plus couvrante et/ou pour favoriser la régénération folliculaire.

14. Composition dermatologique selon l'une quelconque des revendications 7 à 12, pour son utilisation dans la prévention et/ou le traitement de l'alopécie.

15. Procédé de soin non-thérapeutique des cheveux destiné à améliorer l'esthétique des cheveux en favorisant la pousse capillaire et/ou à obtenir une chevelure plus couvrante, **caractérisé en ce qu'**il consiste à appliquer sur les cheveux et le cuir chevelu une quantité efficace d'une association comprenant un extrait de quinquina, un extrait de *Leontopodium alpinum* et du sel de manganèse de l'acide carboxylique L-pyrrolidone selon l'une quelconque des revendications 1 à 3 ou d'une composition cosmétique selon l'une quelconque des revendications 7 à 12, à laisser celle-ci au contact des cheveux et du cuir chevelu, et éventuellement à rincer les cheveux et le cuir chevelu.

## Patentansprüche

1. Kombination, umfassend einen Extrakt aus Chinarinde, einen Extrakt aus *Leontopodium alpinum* und Mangansalz der L-Pyrrolidoncarbonsäure.

2. Kombination nach Anspruch 1, **dadurch gekennzeichnet, dass** der Extrakt aus Chinarinde ein Rindenextrakt aus roter Chinarinde ist.

3. Kombination nach einem der Ansprüche 1 und 2, **dadurch gekennzeichnet, dass** der Extrakt aus *Leontopodium alpinum* ein hydroalkoholischer Extrakt ist.

4. Kombination nach einem der Ansprüche 1 bis 3 für ihre Verwendung zur Vorbeugung und/oder zur Behandlung des Haarausfalls.

5. Kombination für ihre Verwendung nach Anspruch 4, wobei der Haarausfall aus der Gruppe ausgewählt ist, die aus dem androgenetischen Haarausfall, dem reaktiven Haarausfall, dem Haarausfall in der Postmenopause und dem kreisrunden Haarausfall besteht.

6. Nicht-therapeutische Verwendung der Kombination nach einem der Ansprüche 1 bis 3 zur Begrenzung des Ausfalls der Haare und/oder des Fells und/oder zur Förderung des Haarwachstums und/oder zur Steigerung der Dichte der Haarfollikel und/oder um ein dichteres Haar zu erhalten und/oder um die Regenerierung der Follikel zu fördern.

7. Kosmetische oder dermatologische Zusammensetzung, umfassend als Wirkstoff eine Kombination nach einem der Ansprüche 1 bis 3 mit mindestens einem kosmetisch oder dermatologisch akzeptablen Hilfsstoff.

8. Zusammensetzung nach Anspruch 7, **dadurch gekennzeichnet, dass** sie 0,01 % bis 10 %, insbesondere 0,5 % bis 6 %, vorzugsweise 1 % bis 5 Gew.-% eines Extrakts aus Chinarinde im Verhältnis zum Gesamtgewicht der Zusammensetzung umfasst.

9. Zusammensetzung nach einem der Ansprüche 7 und 8, **dadurch gekennzeichnet, dass** sie 0,01 % bis 1,5 %, insbesondere 0,01 % bis 1,0 %, vorzugsweise 0,05 % bis 0,5 Gew.-% Mangansalz der L-Pyrrolidoncarbonsäure im Verhältnis zum Gesamtgewicht der Zusammensetzung umfasst.

10. Zusammensetzung nach einem der Ansprüche 7 bis 9, **dadurch gekennzeichnet, dass** sie 0,01 % bis 5 %, insbesondere 0,05 % bis 2 %, vorzugsweise 0,1 % bis 1 Gew.-% eines Extrakts aus *Leontopodium alpinum* im Verhältnis zum Gesamtgewicht der Zusammensetzung umfasst.

11. Zusammensetzung nach einem der Ansprüche 7 bis 10, **dadurch gekennzeichnet, dass** sie ferner einen anderen Wirkstoff gegen Haarausfall umfasst.

12. Zusammensetzung nach einem der Ansprüche 7 bis 11, **dadurch gekennzeichnet, dass** sie in einer Form vorliegt, die für eine topische Anwendung geeignet ist.

13. Nicht-therapeutische Verwendung einer kosmetischen Zusammensetzung nach einem der Ansprüche 7 bis 12 zur Begrenzung des Ausfalls der Haare und/oder des Fells und/oder zur Förderung des Haarwachstums und/oder zur Steigerung der Dichte der Haarfollikel und/oder um ein dichteres Haar zu erhalten und/oder um die Regenerierung der Follikel zu fördern.

14. Dermatologische Zusammensetzung nach einem der Ansprüche 7 bis 12 für ihre Verwendung zur Vorbeugung und/oder zur Behandlung des Haarausfalls.

15. Nicht-therapeutisches Haarpflegeverfahren, das zur Verbesserung der Ästhetik der Haare bestimmt ist, durch Förderung des Haarwachstums und/oder Erhalt eines dichteren Haars, **dadurch gekennzeichnet, dass** es darin besteht, auf das Haar und die Kopfhaut eine wirksame Menge einer Kombination aufzutragen, die einen Extrakt aus Chinarinde, einen Extrakt aus *Leontopodium alpinum* und Mangansalz der L-Pyrrolidoncarbonsäure nach einem der Ansprüche 1 bis 3 oder eine kosmetische Zusammensetzung nach einem der Ansprüche 7 bis 12 umfasst, diese im Kontakt mit den Haaren und der Kopfhaut zu lassen und eventuell das Haar und die Kopfhaut abzuspülen.

## Claims

1. A combination comprising an extract of cinchona, an extract of *Leontopodium alpinum,* and the manganese salt of L-pyrrolidone carboxylic acid.

2. The combination according to claim 1, **characterized in that** the extract of cinchona is an extract of red cinchona bark.

3. The combination according to any one of claims 1 and 2, **characterized in that** the extract of *Leontopodium alpinum* is a hydroalcoholic extract.

4. The combination according to any one of claims 1 to 3, for use in the prevention and/or treatment of alopecia.

5. The combination for use according to claim 4 wherein the alopecia is selected from the group consisting of androgenetic alopecia, reactive alopecia, postmenopausal alopecia and alopecia areata.

6. A cosmetic use of the combination according to any one of claims 1 to 3, to limit head and/or body hair loss and/or to promote hair growth and/or to increase hair follicle density and/or to obtain hair with greater coverage and/or to promote follicular regeneration.

7. A cosmetic or dermatological composition comprising as active principle a combination according to any one of claims 1 to 3, with at least one cosmetically or dermatologically acceptable excipient.

8. The composition according to claim 7, **characterized in that** it comprises 0.01% to 10%, in particular 0.5% to 6%, preferably 1% to 5%, by weight of an extract of cinchona relative to the total weight of the composition.

9. The composition according to any one of claims 7 and 8, **characterized in that** it comprises 0.01% to 1.5%, in particular 0.01% to 1.0%, preferably 0.05% to 0.5%, by weight of the manganese salt of L-pyrrolidone carboxylic acid relative to the total weight of the composition.

10. The composition according to any one of claims 7 to 9, **characterized in that** it comprises 0.01% to 5%, in particular 0.05% to 2%, preferably 0.1% to 1%, by weight of an extract of *Leontopodium alpinum* relative to the total weight of the composition.

11. The composition according to any one of claims 7 to 10, **characterized in that** it further comprises another anti-alopecia active principle.

12. The composition according to any one of claims 7 to 11, **characterized in that** it is in a form suitable for topical application.

13. A cosmetic use of a cosmetic composition according to any one of claims 7 to 12 for limiting head and/or body hair loss and/or for promoting hair growth and/or for increasing hair follicle density and/or for obtaining hair with greater coverage and/or for promoting follicular regeneration.

14. The dermatological composition according to any one of claims 7 to 12, for use in the prevention and/or treatment of alopecia.

15. A cosmetic hair care process intended to improve the aesthetics of the hair by promoting hair growth and/or to obtain hair with greater coverage, **characterized in that** it consists in applying to the hair and the scalp an effective amount of a combination comprising an extract of cinchona, an extract of *Leontopodium alpinum* and the manganese salt of L-pyrrolidone carboxylic acid according to any one of claims 1 to 3 or a cosmetic composition according to any one of claims 7 to 12, leaving the latter in contact with the hair and scalp, and optionally rinsing the hair and scalp.
